# EUROPEAN PATENT SPECIFICATION

(11) **EP 3 134 067 B1**
(45) Date of publication and mention of the grant of the patent: **27.11.2024**
(21) Application number: 14828049.8
(22) Date of filing: 12.12.2014
(51) Int. Cl.: A61K 9/00, A61K 31/23, A61K 31/695, A61K 31/80, A61P 33/14, A01N 37/02, A01P 7/04

(54) **VETERINARY COMPOSITION**
VETERINÄRMEDIZINISCHE ZUSAMMENSETZUNG
COMPOSITION VÉTÉRINAIRE

(30) Priority: 22.04.2014 GB 201407051
(43) Date of publication of application: 01.03.2017
(73) Proprietor: Pets Choice Limited, Blackburn, Lancashire BB1 5UD (GB)
(72) Inventor: SPURLOCK, Mark, Frome Somerset BA11 2JJ (GB)
(74) Representative: Greaves Brewster LLP
(86) International application number: PCT/GB2014/053691
(87) International publication number: WO 2015/162395

(56) References cited:
- WO-A1-01/19190
- WO-A1-2013/030768
- US-A1- 2003 202 997
- "Encyclopedia of Emulsion Technology Applications", 1 January 1983, TAYLOR & FRANCIS, article DAVIES S. STANLEY ET AL: "Medical and Pharmaceutical Applications of Emulsions", pages: 210 - 210, XP093021575
- DATABASE GNPD [online] MINTEL; 14 September 2006 (2006-09-14), ANONYMOUS: "Head Lice Treatment", XP093069369, retrieved from https://www.gnpd.com/sinatra/recordpage/587032/ Database accession no. 587032
- JORG HEUKELBACH ET AL: "Dimeticone-Based Pediculicides: A Physical Approach to Eradicate Head Lice", THE OPEN DERMATOLOGY JOURNAL, vol. 4, no. 1, 1 January 2010 (2010-01-01), pages 77 - 81, XP055171059, ISSN: 1874-3722, DOI: 10.2174/1874372201004010077

## Description

The present invention relates to a veterinary composition for use in the treatment of flea infestation in a pet, comprising a combination of dimethicone and cyclomethicone as defined in the claims.

Many animals have parasites that live on the surface of their bodies, such as lice, fleas and ticks. These parasites bite the animal's skin and ingest its blood, often injecting foreign proteins with anticoagulant properties. This can irritate the animal, initiate allergic responses and may cause infections.

These parasites can have a negative effect on any people the animal comes into contact with, or environments that the animal frequents. This issue is of greater significance when the animal is domesticated and lives in close contact with its owner, and in particular in the case of fleas and ticks. Lice are species specific and generally spend their entire lives linked to a single host. By contrast, fleas and ticks move from one host to another, whether they are humans or animals and hence capable of spreading diseases from one host to another.

A number of solutions have been proposed to repel or eradicate parasites and conventional treatments include the application of a parasiticidal pesticide to the pet. However, there is a growing demand for pesticide-fee products over environmental concerns and potential issues of toxicity of the pesticide to the pet. However, it has been observed that most of the natural treatments currently available have lower efficacy than pesticide-containing products.

US 2003/202997 A1 describes compositions and methods for killing ectoparasites on a subject. Compositions containing a fatty acid ester, e.g., isopropyl myristate, effective for killing ectoparasites are described. Also described are compositions containing a fatty acid ester and a siloxane (e.g. decacyclomethicone). The compositions can also contain a mectin and/or a mycin, and S-methoprene.

It is an object of this invention to mitigate problems such as those described above, or to provide an alternative to existing products.

According to a first aspect of the invention, there is provided a veterinary composition comprising dimethicone, cyclomethicone and isopropyl myristate for use in the treatment of flea infestation in a pet, wherein dimethicone is present in an amount of from 2 %w/v to 20 %w/v, cyclomethicone is present in an amount of from 10 %w/v to 40 %w/v, and isopropyl myristate is present in an amount of from 20 %w/v to 70 %w/v, and wherein the composition further comprises benzyl alcohol, wherein benzyl alcohol is present in an amount of from 2 %w/v to 15%w/v.

The invention provides a pesticide-free, environmentally friendly and low toxicity treatment for parasite infestation. It has been observed that this composition is particularly efficient for the treatment of flea infestation in pets, such as dogs and cats.

Dimethicone is present in an amount of from 2 %w/v to 20 %w/v. The value ranges provided in this application are inclusive of the upper and lower limits. The preferred viscosity of the dimethicone is 100 centistokes, as this has proven skin compatibility in a wide range of topical formulations, low volatility and lends itself to formulation in mobile liquid preparations.

Hair products for the treatment of lice infestation in humans are commercially available, which comprise dimethicone. Dimethicone coats the lice and disrupts its ability to excrete water. The effect on flea differs in that dimethicone acts as a sticky trap that immobilises the flea, thereby preventing it from biting. This mechanism is particularly advantageous for the treatment of flea infestation as it prevents fleas from migrating to another host. Thus, the flea infestation is stopped in the host and migration to other hosts is prevented. Moreover, this mechanism is advantageous in that it would be difficult to imagine how fleas could develop a resistance to this mode of action, as opposed to other treatments involving a chemical and/or biological mechanism.

Cyclomethicone is present in an amount of from 10 %w/v to 40 %w/v. In the hair products mentioned above, dimethicone is sometimes provided in an inert cyclomethicone solvent carrier. It has been discovered that the combination of dimethicone with cyclomethicone has an unexpected effect when used in the treatment of flea infestation/pets whereby the rapid enhancement of the skin surface spreading of the dimethicone by the inclusion of cyclomethicone reduces the time to immobilisation of the fleas, i.e. enhancing the speed of efficacy. This is particularly important when the formulation is applied to the recognised predilection sites for fleas on cats and dogs, i.e. the regions where their removal by the routine grooming behaviour of the animal would be least effective, typically the skin along the mid-dorsal line, the base of the tail and the base of the neck.

The use of an inert cyclomethicone solvent carrier may well be suitable for the treatment of lice infestation in humans where only a small surface area is to be covered, but would be unsuitable for the treatment of animals. for example via spot-on formulation where residual efficacy is desired. The isopropyl myristate facilitates the migration of the dimethicone into the skin surface lipids, both enhancing the skin surface spread of the dimethicone but also enabling the dimethicone to form a reservoir within the pool of skin surface lipids, including the sebaceous gland secretions; from these reservoirs the dimethicone is presented to the skin surface (and therefore to the fleas that reside there) providing an appropriate residuality and facilitating protracted intervals between re-treatment. Thus, the present composition comprises isopropyl myristate in an amount of from 20 %w/v to 70 %w/v.

The veterinary composition further comprises benzyl alcohol. Benzyl alcohol is present in an amount of from 2 %w/v to 15 %w/v. Benzyl alcohol is added as a co-solvent to the composition to facilitate the formulation of the product, since the combining of the dimethicone with the other ingredients is problematic in the context of forming a uniform blend that does not undergo phase separation. Benzyl alcohol is the preferred alcohol in this context as it has well established and documented use in a wide range of veterinary spot-on formulations and therefore should not present any unusual toxicity or skin intolerance hazards for the target species for the preferred formulation.

A preferred veterinary composition according to the present invention consists of dimethicone, cyclomethicone, isopropyl myristate and benzyl alcohol. The most preferred composition consists of 10%w/v dimethicone, 20%w/v cyclomethicone, 9.8 %w/v benzyl alcohol, and the complement to 100%w/v of isopropyl myristate.

The present veterinary composition is preferably applied as a topical solution, with no systematic absorption required to achieve efficacy.

According to a second aspect of the present invention, there is provided a spot-on device comprising a veterinary composition as described above and defined in the claims.

Most products using the immobilisation of the parasite as a mode of action requires substantial amounts of formulation, which is sprayed onto the whole animal or used as a lotion or shampoo. This can be expensive to produce, package and transport and increase, in addition, the potential toxicity is increased because the formulation is applied to the whole of the animal, which will then lick its fur and ingest the formulation. It is therefore important to rinse off or wash off the formulation after application. Moreover, some components, such as benzyl alcohol in large amounts have been found to have a negative effect (i.e. melting) on some plastic materials and it is therefore essential to wait for the fur of the treated pet to be dry before letting the animal in contact with such materials. The challenge was therefore to find a composition which could be administered in small amounts and suitably migrate over the pet's skin surface to cover the whole area to be treated, and still be efficient in immobilising the parasites.

The present veterinary composition has been found to be particularly advantageous when delivered from a spot-on device or pipette. A small accurate amount of composition is applied to the animal, preferably at a location that the animal is not able to reach (e.g. the back of the neck). The specific combination of ingredients enables the efficient migration of the composition over the whole surface to be treated and still enables the immobilisation of the parasites. Furthermore, it has been observed that the physical properties of the present composition are such that an accurate dosage can be expelled from the spot-on device.

Preferably, the spot-on device comprises 1.5 ml of veterinary composition for the treatment of a dog or 0.7 ml of veterinary composition for the treatment of a cat.

According to a third aspect of the present invention, there is provided method for the preparation of a veterinary composition as defined in the claims, comprising the steps of (a) mixing dimethicone with cyclomethicone; (b) adding a portion of the total amount of isopropyl myristate and mixing; (c) adding benzyl alcohol and mixing; and (d) adding the remainder of the isopropyl myri state.

The specific sequence of the process of manufacture is important because it ensures uniform and intimate mixing of the individual components and minimises the risk of phase separation, particularly of the dimethicone.

The present invention provides a veterinary composition as described above for use in the treatment of flea infestation in a pet. Preferably, the pet is a dog or a cat.

The veterinary composition according to the present invention is prepared by blending together cyclomethicone and dimethicone in the required amounts. The bulk of the isopropyl myristate is added to the blend and thoroughly mixed, Benzyl alcohol is added and the blend is mixed well until a clear solution is obtained. Isopropyl myristate is added to make up to the final volume and mixed. An integrally moulded spot-on device is provided, filled with the veterinary composition and sealed close.

In use, a spot-on device containing a veterinary composition according to the present invention is provided. When required, the user removed the cap of the spot-on and directs the aperture towards the animal's skin (preferably at the back of the neck where the animal cannot lick). The composition is released onto the skin by squeezing the pipette until the spot-on is substantially empty. The composition migrates across the animal skin via passive dispersion in the sebum to treat the infestation. The fleas are immobilised by the composition which acts as a sticky trap, thereby rendering the fleas inactive and unable to migrate to another host.

A preferred veterinary composition according to the present invention has the following formulation.

| **Ingredient** | **%w/v** |
|---|---|
| Cyclomethicone 5 (BC VS5 Silicone Fluid) | 20.0 |
| Dimethicone 100cS liquid | 10.0 |
| Benzyl alcohol | 9.80 |
| Isopropyl Myristate | To 100.0 |
| Total | 100.0 |

Excellent efficacy was observed with all adult fleas in an in vitro exposure test (using concentrations of product likely to be found on the skin surface of a treated animal) rendered non-viable within 24 to 48 hours.

Thus, from the above description, it can be seen that the present invention provides a pesticide-free veterinary composition for the treatment of flea infestation. The composition offers an efficient natural alternative to existing treatments, combined with a low toxicity to the animal receiving the treatment.

## Claims

1. A veterinary composition comprising dimethicone and cyclomethicone and isopropyl myristate for use in the treatment of flea infestation in a pet, wherein dimethicone is present in an amount of from 2 %w/v to 20 %w/v, cyclomethicone is present in an amount of from 10 %w/v to 40 %w/v, and isopropyl myristate is present in an amount of from 20 %w/v to 70 %w/v, and wherein the composition further comprises benzyl alcohol, wherein benzyl alcohol is present in an amount of from 2 %w/v to 15%w/v.

2. The composition for use according to claim 1 wherein benzyl alcohol is present in an amount of from 2 %w/v to 10%w/v.

3. The composition for use according to claim 1 or 2 comprising 10%w/v dimethicone 100cS liquid, 20%w/v cyclomethicone 5, 9.8 %w/v benzyl alcohol, and the complement to 100%w/v of isopropyl myristate.

4. The composition for use according to any preceding claim wherein the pet is a dog or a cat.

5. A spot-on device comprising a veterinary composition comprising dimethicone and cyclomethicone and isopropyl myristate, wherein dimethicone is present in an amount of from 2 %w/v to 20 %w/v, cyclomethicone is present in an amount of from 10 %w/v to 40 %w/v, and isopropyl myristate is present in an amount of from 20 %w/v to 70 %w/v, and wherein the composition further comprises benzyl alcohol, wherein benzyl alcohol is present in an amount of from 2 %w/v to 15%w/v, for the treatment of flea infestation in a pet.

6. The spot-on device according to claim 5 comprising 1.5 ml of the veterinary composition for the treatment of a dog.

7. The spot-on device according to claim 5 comprising 0.7m1 of the veterinary composition for the treatment of a cat.

8. A method for the preparation of a veterinary composition comprising dimethicone and cyclomethicone and isopropyl myristate, wherein dimethicone is present in an amount of from 2 %w/v to 20 %w/v, cyclomethicone is present in an amount of from 10 %w/v to 40 %w/v, and isopropyl myristate is present in an amount of from 20 %w/v to 70 %w/v, and wherein the composition further comprises benzyl alcohol, wherein benzyl alcohol is present in an amount of from 2 %w/v to 15%w/v, for the treatment of flea infestation in a pet, wherein the method comprises the steps of (a) mixing dimethicone with cyclomethicone; (b) adding a portion of the total amount of isopropyl myristate and mixing; (c) adding benzyl alcohol and mixing; and (d) adding the remainder of the isopropyl myristate.

## Patentansprüche

1. Veterinärmedizinische Zusammensetzung, die Dimethicon und Cyclomethicon und Isopropylmyristat umfasst, zur Verwendung bei der Behandlung eines Flohbefalls bei einem Haustier, wobei Dimethicon in einer Menge von 2 % Gew./Vol. bis 20 % Gew./Vol. vorhanden ist, Cyclomethicon in einer Menge von 10 % Gew./Vol. bis 40 % Gew./Vol. vorhanden ist und Isopropylmyristat in einer Menge von 20 % Gew./Vol. bis 70 % Gew./Vol. vorhanden ist, und wobei die Zusammensetzung ferner Benzylalkohol umfasst, wobei Benzylalkohol in einer Menge von 2 % Gew./Vol. bis 15 % Gew./Vol. vorhanden ist.

2. Zusammensetzung zur Verwendung nach Anspruch 1, wobei Benzylalkohol in einer Menge von 2 % Gew./Vol. bis 10 % Gew./Vol. vorhanden ist.

3. Zusammensetzung zur Verwendung nach Anspruch 1 oder 2, umfassend 10 % Gew./Vol. Dimethicon 100cS flüssig, 20 % Gew./Vol. Cyclomethicon 5, 9,8 % Gew./Vol. Benzylalkohol und die Ergänzung zu 100 % Gew./Vol. Isopropylmyristat.

4. Zusammensetzung zur Verwendung nach einem vorhergehenden Anspruch, wobei das Haustier ein Hund oder eine Katze ist.

5. Spot-on-Vorrichtung, umfassend eine veterinärmedizinische Zusammensetzung, die Dimethicon und Cyclomethicon und Isopropylmyristat umfasst, wobei Dimethicon in einer Menge von 2 % Gew./Vol. bis 20 % Gew./Vol. vorhanden ist, Cyclomethicon in einer Menge von 10 % Gew./Vol. bis 40 % Gew./Vol. vorhanden ist und Isopropylmyristat in einer Menge von 20 % Gew./Vol. bis 70 % Gew./Vol. vorhanden ist, und wobei die Zusammensetzung ferner Benzylalkohol umfasst, wobei Benzylalkohol in einer Menge von 2 % Gew./Vol. bis 15 % Gew./Vol. vorhanden ist, zur Behandlung eines Flohbefalls bei einem Haustier.

6. Spot-On-Vorrichtung nach Anspruch 5, umfassend 1,5 ml der veterinärmedizinischen Zusammensetzung zur Behandlung eines Hundes.

7. Spot-On-Vorrichtung nach Anspruch 5, umfassend 0,7 ml der veterinärmedizinischen Zusammensetzung zur Behandlung einer Katze.

8. Verfahren zur Herstellung einer veterinärmedizinischen Zusammensetzung, die Dimethicon und Cyclomethicon und Isopropylmyristat umfasst, wobei Dimethicon in einer Menge von 2 % Gew./Vol. bis 20 % Gew./Vol. vorhanden ist, Cyclomethicon in einer Menge von 10 % Gew./Vol. bis 40 % Gew./Vol. vorhanden ist und Isopropylmyristat in einer Menge von 20 % Gew./Vol. bis 70 % Gew./Vol. vorhanden ist, und wobei die Zusammensetzung ferner Benzylalkohol umfasst, wobei Benzylalkohol in einer Menge von 2 % Gew./Vol. bis 15 % Gew./Vol. vorhanden ist, zur Behandlung eines Flohbefalls bei einem Haustier, wobei das Verfahren die Schritte (a) Mischen von Dimethicon mit Cyclomethicon; (b) Hinzufügen eines Teils der Gesamtmenge an Isopropylmyristat und Mischen; (c) Hinzufügen von Benzylalkohol und Mischen; und (d) Hinzufügen des restlichen Isopropylmyristats umfasst.

## Revendications

1. Composition vétérinaire comprenant de la diméthicone et de la cyclométhicone et du myristate d'isopropyle, à utiliser dans le traitement d'une infestation par les puces chez un animal domestique, dans laquelle la diméthicone est présente en une quantité de 2 % p/v à 20 % p/v, la cyclométhicone est présente en une quantité de 10 % p/v à 40 % p/v, et le myristate d'isopropyle est présent en une quantité de 20 % p/v à 70 % p/v, et dans laquelle la composition comprend en outre de l'alcool benzylique, dans lequel l'alcool benzylique est présent en une quantité de 2 % p/v à 15 % p/v.

2. Composition à utiliser selon la revendication 1, dans laquelle l'alcool benzylique est présent en une quantité de 2 % p/v à 10 % p/v.

3. Composition à utiliser selon la revendication 1 ou 2 comprenant 10 % p/v de diméthicone 100cS liquide, 20 % p/v de cyclométhicone 5, 9,8 % p/v d'alcool benzylique et le complément à 100 % p/v de myristate d'isopropyle.

4. Composition à utiliser selon l'une quelconque des revendications précédentes, dans laquelle l'animal domestique est un chien ou un chat.

5. Dispositif d'application en un point, comprenant une composition vétérinaire comprenant de la diméthicone et de la cyclométhicone et du myristate d'isopropyle, dans lequel la diméthicone est présente en une quantité de 2 % p/v à 20 % p/v, la cyclométhicone est présente en une quantité de 10 % p/v à 40 % p/v, et le myristate d'isopropyle est présent en une quantité de 20 % p/v à 70 % p/v, et dans lequel la composition comprend en outre de l'alcool benzylique, dans lequel l'alcool benzylique est présent en une quantité de 2 % p/v à 15 % p/v, pour le traitement d'une infestation par les puces chez un animal domestique.

6. Dispositif d'application en un point selon la revendication 5, comprenant 1,5 ml de la composition vétérinaire pour le traitement d'un chien.

7. Dispositif d'application en un point selon la revendication 5, comprenant 0,7 ml de la composition vétérinaire pour le traitement d'un chat.

8. Procédé permettant la préparation d'une composition vétérinaire comprenant de la diméthicone et de la cyclométhicone et du myristate d'isopropyle, dans lequel la diméthicone est présente en une quantité de 2 % p/v à 20 % p/v, la cyclométhicone est présente en une quantité de 10 % p/v à 40 % p/v, et le myristate d'isopropyle est présent en une quantité de 20 % p/v à 70 % p/v, et dans lequel la composition comprend en outre de l'alcool benzylique, dans lequel l'alcool benzylique est présent en une quantité de 2 % p/v à 15 % p/v, pour le traitement d'une infestation par les puces chez un animal domestique, dans lequel le procédé comprend les étapes de (a) mélange de la diméthicone avec de la cyclométhicone ; (b) ajout d'une partie de la quantité totale de myristate d'isopropyle et mélange ; (c) ajout de l'alcool benzylique et mélange ; et (d) ajout du reste du myristate d'isopropyle.
